# EUROPEAN PATENT SPECIFICATION

(11) **EP 4 192 344 B1**
(45) Date of publication and mention of the grant of the patent: **11.02.2026**
(21) Application number: 21856561.2
(22) Date of filing: 10.08.2021
(51) Int. Cl.: A61B 5/022, A61B 5/021, A61B 5/024, A61B 5/026, A61B 17/135, A61B 5/11, A61B 5/00, A61B 5/029, A61B 5/0205, A61B 5/08

(54) **OPTOMECHANICAL METHOD TO MEASURE ARTERIAL PULSE AND ASSESS CARDIOPULMONARY HEMODYNAMICS**
OPTOMECHANISCHES VERFAHREN ZUR MESSUNG DES ARTERIELLEN PULSES UND ZUR BEURTEILUNG DER KARDIOPULMONALEN HÄMODYNAMIK
PROCÉDÉ OPTOMÉCANIQUE DE MESURE DE POULS ARTÉRIEL ET D'ÉVALUATION DE L'HÉMODYNAMIQUE CARDIOPULMONAIRE

(30) Priority: 10.08.2020 US 202063063482 P
(43) Date of publication of application: 14.06.2023
(73) Proprietor: Dynocardia, Inc., Cambridge, Massachusetts 02142 (US)
(72) Inventor: ADELSON, Edward H., Winchester, Massachusetts 01890 (US); BISWAS, Abhijit, Parganas North 743127 (IN); THANIKACHALAM, Mohan, Newton, Massachusetts 02149 (US)
(74) Representative: Beck Greener LLP
(86) International application number: PCT/US2021/045368
(87) International publication number: WO 2022/035841

(56) References cited:
- WO-A1-2019/195120
- WO-A1-2019/195120
- WO-A1-2020/082133
- WO-A2-2009/155501
- US-A1- 2005 070 775
- US-A1- 2010 094 146
- US-B1- 6 533 729

## Description

The measurement of cardiopulmonary parameters, such as, heart rate (HR), blood pressure (BP), respiratory rate (RR) and respiratory pattern, and other hemodynamic parameters, such as cardiac output, stroke volume and peripheral vascular resistance, is known to be important in human health. In particular, the ability to identify abnormal BP patterns, such as white coat hypertension, masked hypertension, and non-dipping and other abnormal patterns during sleep and morning BP surge can be particularly important. Being able to accurately measure cardiopulmonary parameters in a noninvasive and dynamic manner can facilitate the accurate assessment and treatment of critically ill patients in hospitals. This accurate measurement can allow for physicians and other healthcare personnel to better tailor therapy and/or allow for the identification of lifestyle motivations for management of hypertension and other chronic diseases that benefit form tight BP control, such as, heart failure, sleep apnea and chronic renal failure. Conventional BP and hemodynamic monitoring devices suffer from accuracy issues, do not provide dynamic results over extended periods of time, or are relatively invasive, all of which greatly limit the number of situations in which the devices can be used. Therefore, systems that are adapted to monitor a patient's hemodynamic parameters in a noninvasive, dynamic manner for extended periods of time would be highly advantageous. An example of a tactile blood pressure imager is described in WO 2019195120A1. WO2009155501A2 describes a tactile sensor using elastomeric imaging. Other systems are known from WO2020082133A1 and US6533729B.

### SUMMARY

According to aspects of the present invention there is provided, in a first aspect, an optomechanical sensor system for use with a patient according to independent claim 1, and in a second aspect, a method of monitoring a cardiopulmonary parameter of a patient according to independent claim 12.

Embodiments generally describes systems and methods for the noninvasive estimation and continuous monitoring of cardiopulmonary parameters, particularly, via directly measuring skin surface displacements and forces over superficial arteries, such as the radial artery at the wrist.

### FIGURES

The accompanying drawings, which are incorporated in and form a part of the specification, illustrate the embodiments of the invention and together with the written description serve to explain the principles, characteristics, and features of the invention. In the drawings:
FIG. 1 A is a block diagram of a noninvasive hemodynamic parameter monitoring system, in accordance with an embodiment of the present disclosure.
FIG. 1B is another block diagram of a noninvasive hemodynamic parameter monitoring system, in accordance with an embodiment of the present disclosure.
FIG. 2 is a diagram of a first embodiment of a noninvasive hemodynamic parameter monitoring system using a deformable gel elastomer, in accordance with an embodiment of the present disclosure.
FIG. 3 is a diagram of a second embodiment of a noninvasive hemodynamic parameter monitoring system using a deformable gel elastomer, in accordance with an embodiment of the present disclosure.
FIG. 4 is a diagram of a first embodiment of a noninvasive hemodynamic parameter monitoring system using a deformable surface, in accordance with an embodiment of the present disclosure.
FIG. 5 is a diagram of a second embodiment of a noninvasive hemodynamic parameter monitoring system using a deformable surface, in accordance with an embodiment of the present disclosure.
FIG. 6A is a diagram of a second embodiment of a noninvasive hemodynamic parameter monitoring system using a piston, in accordance with an embodiment of the present disclosure.
FIG. 6B is a diagram of a second embodiment of a noninvasive hemodynamic parameter monitoring system using a secondary inflatable bladder, in accordance with an embodiment of the present disclosure.
FIG. 7A is a perspective view of the measurement device, in accordance with an embodiment of the present disclosure.
FIG. 7B is a reverse perspective view of the measurement device of FIG. 7A, in accordance with an embodiment of the present disclosure.
FIG. 8A is a perspective view of the measurement device worn by an individual, in accordance with an embodiment of the present disclosure.
FIG. 8B is a reverse perspective view of the measurement device of FIG. 8A worn by an individual, in accordance with an embodiment of the present disclosure.
FIG. 9 is a coordinate system defined with respect to the deformable surface, in accordance with an embodiment of the present disclosure.
FIG. 10A is a temporal for the embodiment of the measurement depicted shown in FIGS. 2 and 3, in accordance with an embodiment of the present disclosure.
FIG. 10B is a temporal for the embodiment of the measurement depicted shown in FIGS. 4 and 5, in accordance with an embodiment of the present disclosure.
FIG. 11A is a graph of illustrative displacement signal for multiple locations for the embodiment of the measurement device shown in FIGS. 2 and 3, in accordance with an embodiment of the present disclosure.
FIG. 11B is a graph of illustrative displacement signals for multiple locations for the embodiment of the measurement device shown in FIGS. 4 and 5, in accordance with an embodiment of the present disclosure.
FIG. 12A is a surface map of maximum optical marking displacement at multiple locations for the embodiment of the measurement device shown in FIGS. 2 and 3, in accordance with an embodiment of the present disclosure.
FIG. 12B is a surface map of maximum optical marking displacement at multiple locations for the embodiment of the measurement device shown in FIGS. 4 and 5, in accordance with an embodiment of the present disclosure.

### DESCRIPTION

Described herein are systems and methods for optically measuring the motions of the skin surface by placing an optomechanical sensor array and applying counter pressure on the skin over a large superficial artery, such as the radial artery at the wrist, to non-invasively assess arterial pulsation and its characteristics. This information can be used to generate continuous arterial pulse waveform and to measure BP, HR, RR, and other hemodynamic parameters. The intra-arterial pressure wave has a direct relationship to forces on the inner surface of the artery wall and its displacement, which, in turn, affects the deformation and forces imposed on the optomechanical sensor array on the skin surface above, particularly at specific locations, such as the region above a superficial artery, such as the radial artery.

The optical method measures a larger vessel arterial pulsation and its characteristics accurately. Measuring HR with this optical method is more reliable compared to Photoplethysmography (PPG), which is an optical measurement method that is often used for heart rate monitoring purposes. The PPG uses a light source and a photodetector at the surface of skin to measure the volumetric variations of capillary blood circulation. Compared to the optical method described here, PPG signal strength is weak and subject to motion artifacts, environmental temperature, etc.

In this optical method, characteristics of the spatiotemporal pattern due to the skin motions observed above the radial artery during the arterial pulse can be altered by varying the external counter pressure applied to the skin above the artery. The characteristics such as shape, size, and displacement of spatiotemporal pattern will vary depending on the counter pressure being applied and intra-vascular pressure. By analyzing the spatiotemporal patterns it is possible to measure BP continuously, which has been previously described by us in PCT Patent Application Publication No. WO 2019/195120 A1, titled TACTILE BLOOD PRESSURE IMAGER, filed March 29, 2019.

During the arterial pulse, the motion of the skin above the radial artery is primarily in the direction normal to the skin surface, which may also be called the z-axis. It is the purpose of this systems and methods described to measure the motion of the skin surface above the radial artery.

### Noninvasive Hemodynamic Parameter Monitoring

Described herein are systems and methods directed to placing a pattern of optical markings over the surface of a patch of skin connected to an artery by adjoining soft tissues in a non-invasive manner and applying a controlled amount of pressure to ensure a tight interface between the deformable surface on which the optical markings are disposed and the patient's skin. The optical markings can be dots, lines, stripes, grids or any other pattern. Once secured in place, changes in the artery geometry and forces can be detected during a heartbeat cycle based on perturbations or movement of the optical markings as caused by the mechanical pulsation of the artery. The changes correspond to spatiotemporal signals from the artery or in the adjoining soft tissues. The spatiotemporal signals can measured and processed, via a computer system or controller, to determine hemodynamic parameters.

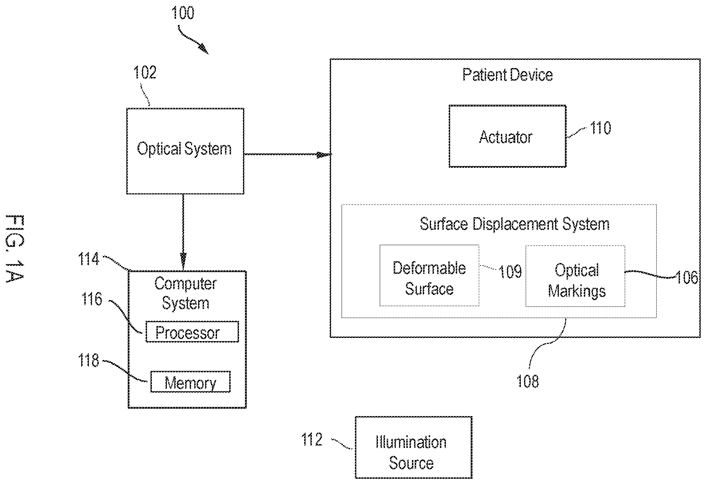

FIG. 1A is a diagram of an embodiment of an optomechanical sensor system 100, in accordance with an embodiment of the present disclosure. The optomechanical sensor system 100 can include an optical system 102 that is configured to visualize or otherwise monitor the patient device. In some embodiments, the optical system 102 can be configured to be detached about the wrist of the patient such that only the patient device or a portion of it (e.g., the surface displacement system 108) bears against the patient's skin adjacent to the radial artery, particularly at the wrist. For example, the surface displacement system 108 can be imaged through a mobile phone optical system completely detached from the patient's body or an optical system attached to a fixed surface (e.g., a physical wall) or some other part of the body (e.g., a shoulder). The surface displacement system 108 can include one or more optical markings 106 positioned on or otherwise associated with a deformable surface 109. The deformable surface 109 can be constructed from a variety of materials, such as thermoplastic polyurethane or nylon. The thickness of the deformable surface 109 can be from, for example, 10 µm to 300 µm.

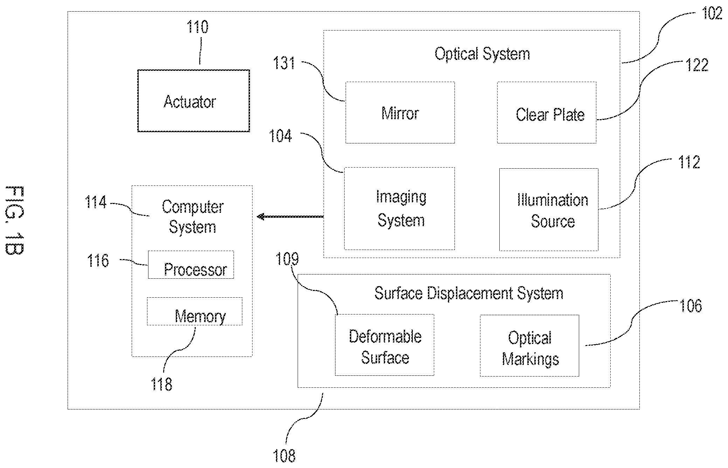

FIG. 1B is a diagram of an optomechanical sensor system 100, in accordance with an embodiment of the present disclosure. The optomechanical sensor system 100 can include an optical system 102, which includes the an imaging system 104 and an illumination source 112. In some embodiments, which are further described below, the optical system 102 may include one or more mirrors 131 and a transparent or clear layer (e.g., a clear plate 122, as described below) through which the imaging system 104 is configured to visualize the surface displacement system 108. In some embodiments, the optical system 102 can be configured to be mounted to or attached about the wrist of the patient such that a portion of the optical system 102 bears against surface displacement system 108 on the patient's skin adjacent to the radial artery, particularly at the wrist. The surface displacement system 108 may include deformable surface 109 with optical markings 106. The optical markings can be dots, lines, stripes, grids or any other pattern. In various embodiments, the deformable surface 109 can include a membrane, an elastomer, an air- or fluid filled bladder, or any combination of these options. As noted above, the deformable surface 108 can be constructed from a variety of materials, such as thermoplastic polyurethane or nylon. The thickness of the deformable surface 108 can be from, for example, 10 µm to 300 µm.

The surface displacement system 108 can be configured to deform, move, or otherwise change in response to mechanical pulsation through the patient's radial artery, thereby causing the optical markings 106 to correspondingly shift. The movement of the optical markings 106 can be visualized via the imaging system 104. The optomechanical sensor system 100 can further include a computer system 114 coupled to the optical system 104. The computer system 114 can be programmed or otherwise configured to calculate one or more cardiopulmonary parameters associated with the patient based on the detected movement of the optical markings 106 visualized via the optical system 104. In some embodiments, the optical system 102 can further include an illumination source 112, which can be configured to illuminate the optical markings 106 with visible light or electromagnetic radiation (EMR) in the nonvisible portion of the spectrum (e.g., infrared or ultraviolet). In some embodiments, the illumination source 112 can also be used to project optical markings on the deformable surface 109.

The optomechanical sensor system 100 can further include an actuator 110 that is configured to cause the surface displacement system 108 to apply force to the skin over the large artery (e.g., radial artery at the wrist) in order to assist in the visualization of the arterial pulsation. In particular, if the interface between the surface displacement system 108 and skin is not tightly coupled, then it could negatively impact the ability of the patient's arterial pulsation to deform the surface displacement system 108, which could in turn affect the ability of the optomechanical system 100 to detect and calculate the patient's cardiorespiratory parameters. The actuator 110 may include a bounded space such as a balloon or a chamber, which may be inflated by an air pump or a motorized piston. In some embodiments, the actuator 110 may include a manually adjusted component, such as a manual strap. In another embodiment, the actuator 110 can include an electromechanically adjusted component, such as a motor configured to tighten a strap, a balloon, a cuff, a bladder, or another such component that is configured to exert pressure against the patient.

The computer system 114 can include hardware, software, firmware, or combinations thereof that are programmed or otherwise configured to cause the computer system 114 to perform the actions described herein. In the illustrated embodiment, the computer system 114 can further include a processor 116 and a memory 118 coupled thereto. The memory 118 can stores instructions that, when executed by the processor 116, cause the computer system 114 to receive, from the imaging system 104, images of the optical markings 106; determine the spatiotemporal movement of the superficial artery (e.g., the radial artery) based on the received plurality of images; and determine cardiopulmonary parameters based thereon. For example, the computer system 114 could reconstruct arterial pulse and measure BP based on respiratory variance of BP. As another example, the computer system 114 could measure RR and respiratory pattern and, based on analysis of BP waveform, measure cardiac output, stroke volume, and other cardiopulmonary parameters. In one embodiment, the computer system 114 could be incorporated into or otherwise integral with the patient-worn device, the imaging system 104, or other components of the optomechanical sensor system 100. In another embodiment, the computer system 114 could be communicably coupled to the patient-worn device, the imaging system 104, or other components of the optomechanical sensor system 100. For example, the computer system 114 could be embodied as a cloud computing architecture.

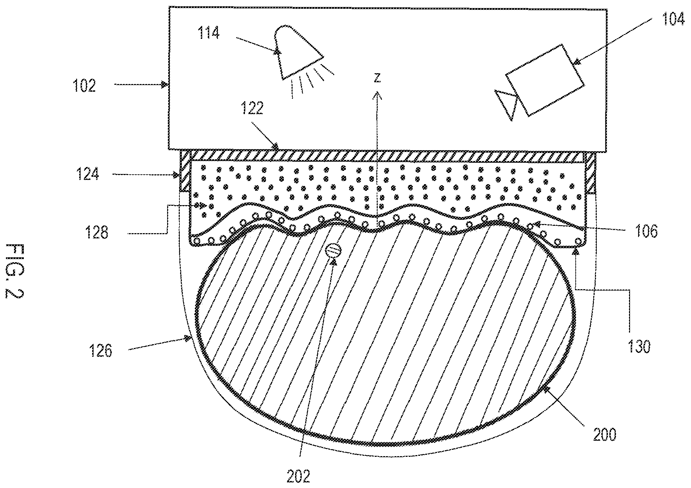

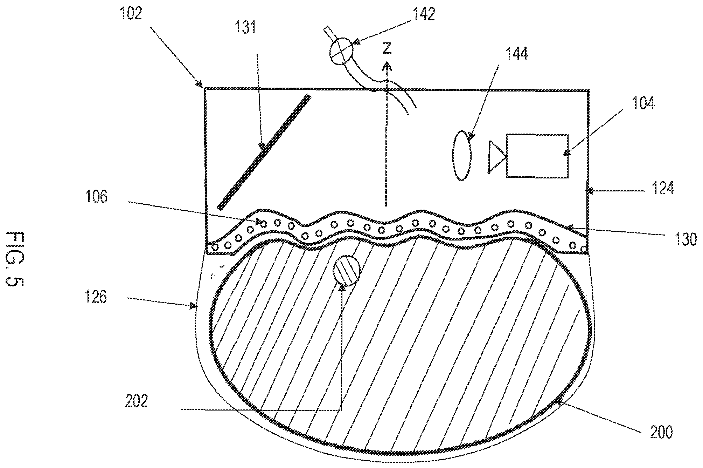

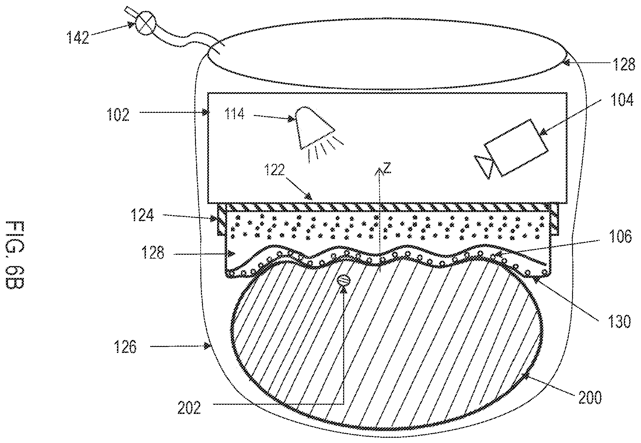

In various embodiments, the optical system 104 can include an individual camera (or an image sensor) or multiple cameras (or image sensors) that can be configured to provide stereoscopic visualization of the optical markings 106. In some embodiments, the optical system 102 can be positioned either above the actuator 110 (as with the embodiments shown in FIGS. 2-4) or below the actuator 110 (as with the embodiments shown in FIG. 6B). In other embodiments, the optical system 10 can be located internally or otherwise integrated into the actuator 110, such as with the embodiments shown in FIG. 5. In any of these embodiments, the imaging system 104 can be positioned at an oblique angle with respect to the surface displacement system 108, thereby increasing the sensitivity of surface displacement detection (Z motions) and improving the visualization of the optical markings 106, as is described in greater detail below.

In various embodiments, the optical markings 106 can include a variety of different colors or patterns that are visually identifiable by the imaging system 104. Further, in various embodiments, the optical markings 106 could be configured to reflect or emit EMR in the visible portion of the EMR spectrum; however, the optical markings 106 could also or additionally be configured to reflect or emit EMR in other portions of the EMR spectrum, such an infrared (IR) or ultraviolet (UV). Accordingly, the imaging system 104 could be configured to utilize a variety of different imaging modalities and include the corresponding components. For example, the optical markings 106 could include IR markings and the imaging system 104 could include an IR camera or image sensor. As another example, the pattern of the optical markings 106 on the deformable surface 109 could include a series of black marks on a white background, white marks on a black background, or any other sets of colors that provide a distinguishable pattern.

One issue that must be overcome is that illuminating light could produce visible artifacts, such as unwanted reflections and scattered light, which could degrade the quality of the image of the optical markings 106 captured by the imaging system 104 and used to measure the patient's arterial pulsation. In order to reduce the visibility of such artifacts, in one embodiment of the optomechanical sensor system 100, the optical markings 106 could include a fluorescent pigment and be illuminated with short wave EMR, such as blue, violet, or UV light. For example, the surface displacement system 108 could include a black deformable surface 109 covered with fluorescent yellow dots as the optical markings 106, which are configured to be illuminated by short wave light. The yellow pattern information will be visible in the red and green channels of a color camera, whereas blue or violet light will be visible in the blue channel. If UV light is used, and the imaging system 104 is insensitive to UV light, then the UV light would not be visible to the imaging system 104.

In another embodiment, the imaging system 104 could include a monochrome camera and the optical markings 106 could include fluorescent patterns. To illustrate, if the exciting illumination is blue light and the fluorescence of the optical markings 106 is yellow, then the blue light can be blocked by placing a yellow filter over the lens of the monochrome camera, thereby ensuring that only the yellow pattern of the optical markings 106 will be visualized. The same principle will work with other pairs of excitation light/fluorescence optical markings 106, where the exciting wavelengths and the emitted wavelengths can be separated through the use of color filters.

Ultraviolet LEDs can be particularly convenient for exciting fluorescence. However, one issue that must be overcome is that most ultraviolet LEDs exhibit "spectral spillage," meaning that there is a small proportion of visible light emitted along with the invisible UV light. To remove this spectral spillage, in one embodiment, the illumination source 112 can include a filter over the LED (e.g., ZWB1, ZWB2, or ZWB3 glass filters) that is configured to reduce or remove the unwanted visible light, thereby mitigating the effects of the LED's spectral spillage.

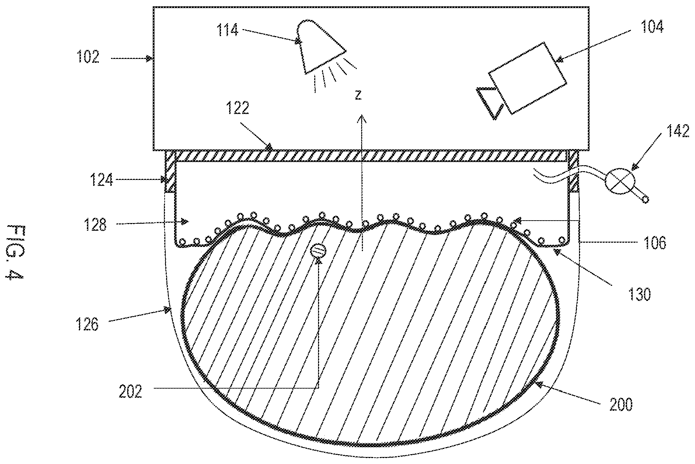

In one embodiment, the deformable surface 109 can include a deformable elastomeric gel 120 and a membrane 130 with optical markings 106 , such as is shown in FIG 2. As noted above, the patient's wrist 200 contains a radial artery 202, which pulsates during the heartbeat. In the depicted embodiment, this pulsation causes motion of the soft tissue and skin above the radial artery 202, which in turn deforms the deformable surface 109, which includes a pad of a deformable elastomeric gel 120 and the membrane 130 optical markings 106. In one embodiment, the deformable elastomeric gel 120 can be clear (i.e., translucent or transparent). The optical system 102 can further include a clear plate 122. The plate can be constructed from polymethyl methacrylate (PMMA) or other such clear materials. In one embodiment, the surface displacement system 108 can be mounted below the clear plate 122 as shown in FIGS. 2 and 4 such that it is configured to be in contact with the patient's skin when worn thereby.

As generally described above, the surface displacement system 108 can include an array of patterns such as dots of various shapes, grids, stripes, and/or various patterns with lines or other optical markings 106. In use, the optical system 102 is mounted over the radial artery 202 at the patient's wrist with a strap 126 attached to the frame 124. The measurement device 102 can be pressed manually against the patient's skin by tightening the strap 126, thereby causing the surface displacement system 108 to conform to the shape of the wrist, including the skin and tissue above the radial artery 202. Further, the contact force between the surface displacement system 108 with the skin can be adjusted by altering the tightness of the strap 126.

As generally described above, the optical system 102 further includes an imaging system 104. In this embodiment, the imaging system 104 can be positioned above the plate 122, such that the imaging system 104 is oriented to visualize the optical markings 106 through the plate 122 and the elastomeric gel 120 and form an image of the optical markings 106. Because the elastomeric gel 120 and plate 122 constructed from clear materials, the imaging system 104 can thus visualize the optical markings 106 disposed along the membrane 130. Further, an illumination source 112 can provide illumination in order to assist in the visualization of the membrane 130 and the corresponding optical markings 106 by the imaging system 104.

Accordingly, when the patient's heart beats, the radial artery 202 expands and contracts, thereby causing the optical markings 106 in contact with the skin to move primarily along the z-axis, as indicated in FIG. 2. As noted above, the imaging system 104 can be oriented at an angle (e.g., an oblique angle) with respect to the optical markings 106. Accordingly, the z-axis motions of the optical markings 106 appear to the imaging system 104 as y-axis motions, i.e., the optical markings 106 appear to move vertically in the plane of the camera sensor. From measurements of the y-axis movements of the optical markings 106 in the captured images, a computer system 114 can calculate the corresponding z-axis motions of the optical markings 106. Accordingly, the spatiotemporal motion of the skin above the radial artery 202 can be determined therefrom. By determining the spatiotemporal motion associated with the radial artery 202, a variety of different cardiopulmonary parameters can be derived therefrom. The placement of the imaging system 104 such that it views the surface displacement system 108 obliquely has two main purposes. The first is to allow for the measurement of z-axis motion, as described above. The second is to allow the device to have a relatively compact form factor, so that it is not too bulky and is convenient to wear on the wrist.

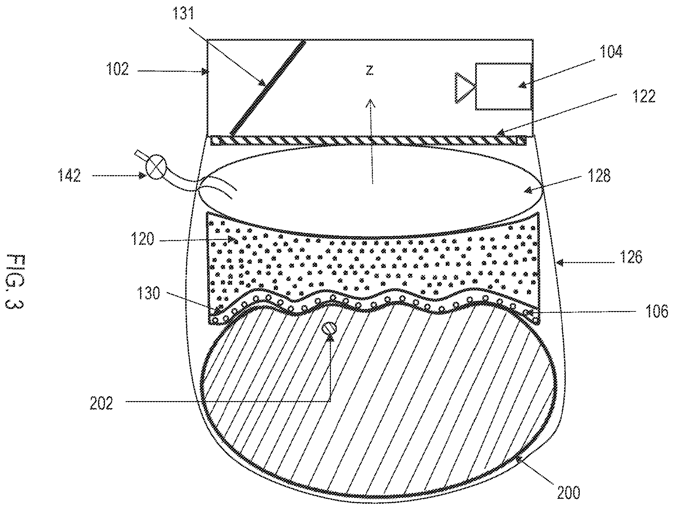

Referring now to FIG. 3, there is shown another embodiment of the optomechanical sensor system 100 that utilizes an elastomeric gel 120. However, this embodiment differs from the embodiment shown in FIG. 2 in that the actuator 110 is a clear inflatable component 128 (e.g., a balloon or bladder) positioned between the elastomeric gel 120 and the plate 122. In this embodiment, the contact force between the surface displacement system 108 with the skin can be adjusted by altering the degree of inflation or air pressure of the inflatable component 128 (i.e., actuator 110) between the plate 122 and the elastomeric gel 120. As with the embodiment shown in FIG. 2, the surface displacement surface 108 conforms to the shape of the wrist, including the skin and tissue above the radial artery 202.

In this embodiment, the optical system 102 further includes a mirror system 131 that is configured to allow the imaging system 104 to indirectly visualize the optical markings 106 therethrough. In another embodiment, the imaging system 104 could be either separate from or integrated directly into the patient-worn components of the optomechanical sensor system 100. The mirror system 131 can include one or multiple mirrors that are configured to provide a view (e.g., at an oblique angle) of the optical markings 106 to the imaging system 104. The optical system 102 can further include one or more lenses 144, such as is shown in the embodiment in FIG. 5.

In one embodiment, the optomechanical sensor system 100 can include an actuator 110 which is an inflatable component 128 (e.g., a balloon or bladder) and the surface displacement system 108, which is the membrane 130 with optical markings 106 such as is shown in FIG 4. This embodiment differs from the embodiments shown in FIGS. 2 and 3 in that the membrane 130 bearing the optical markings 106 is disposed along the inflatable component 128, as opposed to elastomeric gel 120. The inflatable component 128 can be inflated or deflated with fluid i.e., air via a pump 142 operably coupled to the inflatable component 128. The inflatable component 128 can be constructed from a clear elastomeric material, for example. In one embodiment, the optomechanical sensor system 100 can further include a pressure sensor configured to monitor the pressure inside the inflatable component 128 so that a desired pressure or pressure range is maintained. The clear plate 122 and frame 124 in combination with the strap 126 ensures that the inflatable component 128 is held in place against the patient's skin and that it inflates with the surface displacement system 108 oriented towards the patient. In this setup, the contact force between the surface displacement system 108 and the patient's skin can be varied by altering the tightness of the strap 126 and/or varying the pressure in the inflatable component 128. In this embodiment, the imaging system 104 can be positioned to visualize the optical markings 106 through the clear plate 122 and the clear inflatable component 128 to view the optical markings 106 on the deformable surface 108. As with the aforementioned embodiments, the optomechanical sensor system 100 can further include an illumination source 112 to provide illumination for aiding in the visualization of the optical markings 106. As with the aforementioned embodiments, when the patient's heart beats, the radial artery 202 pulses, causing the tissue above it to move. The imaging system 104 can detect the motions of the optical markings 106 and the optomechanical sensor system 100 can correspondingly determine the spatiotemporal motion associated with the radial artery 202, which can in turn be used to derive a variety of different cardiopulmonary parameters associated with the patient.

Referring now to FIG. 5, there is shown another embodiment of the optomechanical sensor system 100 that utilizes an actuator 110, which is a rigid box 125 on all sides except the skin side is covered by surface displacement system 108, and, thus, can be contacted against the patient. In this embodiment, the optical system 102 is positioned within the rigid box 125. Further, the optical system 102 further includes a mirror system 131, similarly to the embodiment shown in FIG. 5, that allows the imaging system 104 to indirectly visualize the optical markings 106 (e.g., at an oblique angle). In this embodiment, the rigid box 125 is connected to a pump 142. The surface displacement system 108 can be pressed against the patient's skin by pumping air in and out of the rigid box 125, thereby causing the surface displacement system 108 to conform to the shape of the wrist, including the skin and tissue above the radial artery 202. Further, the contact force between the surface displacement system 108 with the skin can be adjusted by altering the tightness of the strap 126.

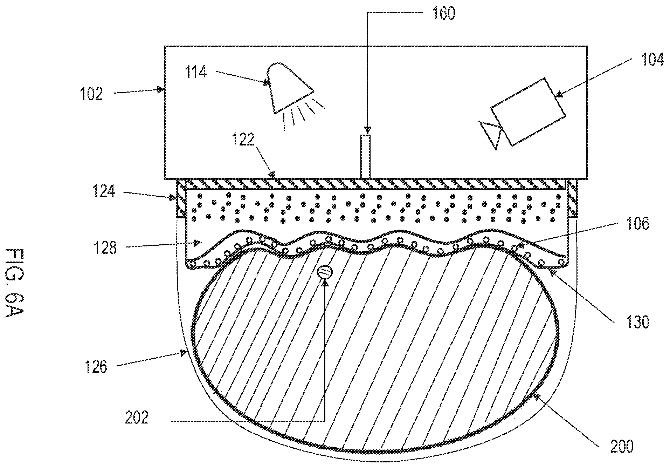

Referring now to FIG. 6A, there is shown another embodiment of the optomechanical sensor system 100 that uses a piston 160. In this embodiment, the surface displacement system 108 includes a deformable surface that is an air or fluid-filled bladder 128 positioned over the membrane 130 with optical markings 106. In addition to a clear plate, this embodiment includes a piston 160 constructed from a clear material (e.g., PMMA) that is configured to be moved (e.g., vertically) with respect to the frame 124. The piston could be made of rigid clear material or a soft bladder that could be inflated / deflated to make it work like a piston. Accordingly, the contact force of surface displacement system 108 with the patient's skin can be varied by altering the tightness of the strap 126, as well as by moving the piston 160. In this embodiment, the imaging system 104 can visualize the optical markings 106 through the clear piston 160. A light source 114 can provide illumination. Once again, when the heart beats, the superficial artery pulses, causing the tissue above it to move, which can be detected by the imaging system 104 based on the motions of the optical markings 106.

In one embodiment shown in FIG. 6B, the optomechanical sensor system 100 can include an actuator 110 which is an inflatable component 128 (e.g., a balloon or bladder) which is between the imaging system 114 and strap 126. The imaging system 114 in turn is in contact with the surface displacement system 108, which is the gel elastomer 120 and the membrane 130 with optical markings 106 such as is shown in FIG 6B. The inflatable component 128 can be inflated or deflated with fluid i.e., air via a pump 142 operably coupled to the inflatable component 128. In this embodiment, the inflatable component 128 can be constructed from an elastomeric material that is durable and need not be as optically clear as the inflatable components in previously described configurations. Some examples of the durable materials could include polyvinyl chloride, thermoplastic polyurethane, and neoprene. In one embodiment, the optomechanical sensor system 100 can further include a pressure sensor configured to monitor the pressure inside the inflatable component 128 so that a desired pressure or pressure range is maintained. In this setup, the contact force between the surface displacement system 108 and the patient's skin can be varied by altering the tightness of the strap 126 and/or varying the pressure in the inflatable component 128. In this embodiment, the imaging system 104 can be positioned to visualize the optical markings 106 on the deformable surface 108 through the clear plate 122 and the clear gel elastomer 120. As with the aforementioned embodiments, the optomechanical sensor system 100 can further include an illumination source 112 to provide illumination for aiding in the visualization of the optical markings 106. As with the aforementioned embodiments, when the patient's heart beats, the radial artery 202 pulses, causing the tissue above it to move. The imaging system 104 can detect the motions of the optical markings 106 and the optomechanical sensor system 100 can correspondingly determine the spatiotemporal motion associated with the radial artery 202, which can in turn be used to derive a variety of different cardiopulmonary parameters associated with the patient.

FIGS. 2-6 show a variety of different embodiments of optomechanical sensor systems 100 for optically measuring z-axis motion with an imaging system 104. In some of these embodiments, the imaging system 104 can include a single camera. In other embodiments, other optical methods, including those using one or more cameras, can be used to measure z-axis position and motion of the optical markings 106. For example, stereopsis, structured light, time-of flight, and other photometric and/or stereo optical techniques could be used to measure z-axis position and motion of the optical markings 106. Many of these methods involve the use of multiple cameras or special optical equipment. However, in some embodiments, it can be beneficial for the optomechanical sensor systems 100 to use optical measurement techniques using only a single camera and with no or minimal special purpose equipment in order to reduce the size, cost, and/or complexity of the optomechanical sensor systems 100.

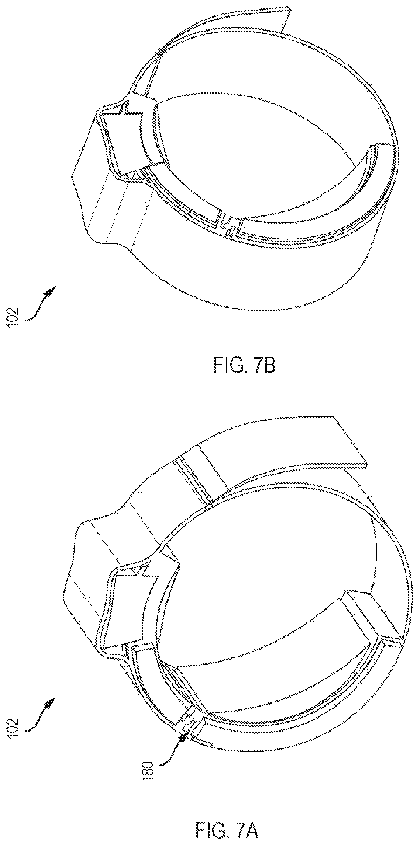

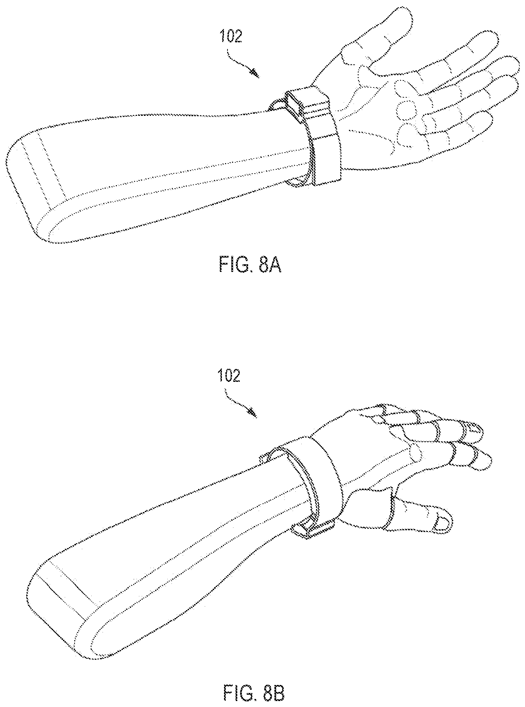

Referring now to FIGS. 7A-8B, there is illustrated an embodiment of the optomechanical sensor system 100 including the various components described above, such as the optical system 102, actuator 110, surface displacement system 108, and the computer system 114 into an illustrative patient-worn device. In one embodiment, the device can be embodied as a half-bracelet having a hinge 180 (FIG. 7A) that can be used to position the optomechanical sensor 100 by using anatomical reference such as radial styloid process. The hinge 180 can also have an adjustable guide to accommodate various wrist sizes. The positioning is accomplished by sliding the device onto the wrist where the hinge 180 or some other mechanism (e.g., a link, an articulated joint, or another adjustable connection) takes the reference of radial styloid process to position the device consistently. The hinge 180 can be then adjusted based on the wrist size and locked in place. In this embodiment, the contact pressure exerted by the surface displacement system108 could be adjusted by (i) compressing the two arms of the bracelet using a motorized system, (ii) adjusting the pressure of an inflatable component 128 (as generally described above, inflating the inflatable component 128 can increase the tension of the strap 126 and thereby increases the contact pressure), and/or (iii) adjusting the strap 126 (e.g., via a manual system, such as a ratchet dial, or an automated system, such as motors that can be used to tighten the strap thus applying the contact force by tensioning the strap). In various embodiments, the actuator 110 and/or surface displacement system 108 can be configured such that the contact pressure exerted by the surface displacement system 108 onto the skin is adjustable from 0 to 350 mmHg.

In some embodiments, the measurement device 102 can be fluid-tight. This could allow, for example, the measurement device 102 to be freely worn by individuals throughout the day, without the need to remove the measurement device 102 to be removed if the individual is performing particular activities (e.g., showering). Therefore, the measurement device 102 could provide uninterrupted monitoring of the patient's hemodynamic parameters.

While the above description pertains to the radial artery 202 of the wrist, this description was simply for illustrative purposes. It will be appreciated that the hemodynamic parameter monitoring system 100 could also be used at other locations on a patient's body and/or with other superficial arteries, such as the carotid artery, temporal artery, anterior tibial artery, posterior tibial artery, or brachial artery. Accordingly, the present disclosure should not be understood to be specifically limited to being used in connection with the patient's wrist and/or the radial artery 202.

### Optical Marking Displacement Analysis

Experimental analysis of various arrangements of the optical markings 106 has identified multiple different candidate patterns and/or imaging system configurations for use with the optomechanical sensor system 100. Further, experimental analysis has determined that the movements of the optical markings 106 can be identified with the requisite specificity and accuracy necessary for tracking the spatiotemporal movements of a superficial artery. The motion analysis of the optical markings 106 can be performed in multiple different ways. For example, an integration method can be used for the motion analysis. In this technique, the cumulative displacement from one frame to the next frame (i.e., the images captured by the imaging system 104) can be continuously calculated. As another example, a reference method can be used for the motion analysis. In this technique, the displacement is calculated relative to a single reference frame. The integration method can offer finer resolution, but could also be prone to accumulated errors over time. Conversely, the reference method can be subject to the brightness constancy conditions which forces it to destabilize under sudden perturbation. Regardless of the motion analysis technique used, the results of the motion analysis are used to obtain the temporal variance of the area of interest.

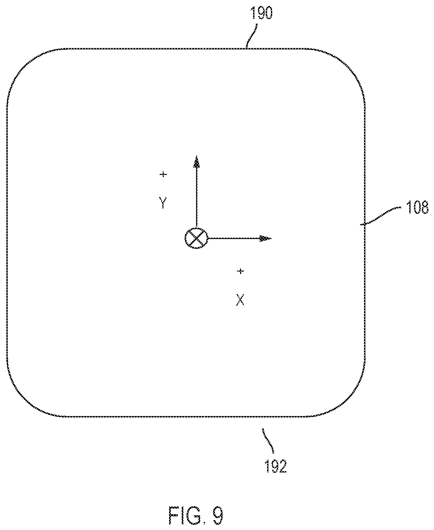

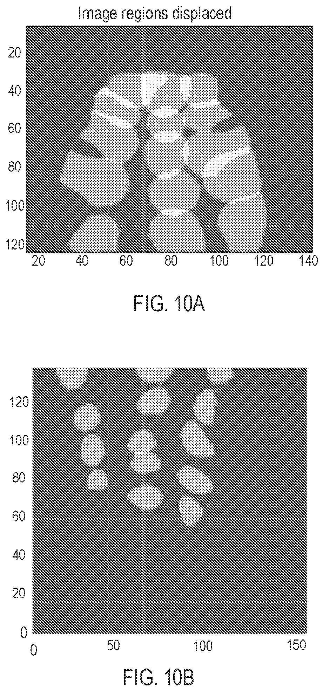

Experimentally, a coordinate system can be defined with respect to the surface displacement system 108, as shown in FIG. 9. Further, a far side 190 and a near side 192 of the surface displacement system 108 can be defined by their proximity to the imaging system 104. In practice, the optical markings 106 will also be more prominent nearer to the imaging system 104 due to the angle at which the imaging system 104 is oriented towards the surface displacement system 108. The center of the surface displacement system 108 can be defined as the origin. Using the defined coordinate system, the temporal mask shown in FIG. 10A was developed for the embodiment shown in FIGS. 2 and 3 and the temporal mask shown in FIG. 10B was developed for the embodiment shown in FIGS. 4 and 5. The temporal maps were generated by thresholding the variance using a predetermined value. By varying the threshold value, different masks can be developed. The area of interest can be determined from the developed maps above, and the height maps obtained from the initial motion analysis can be filtered to obtain data only from the area of interest. The displacement of the optical markings 106 can then be averaged over the entire area of interest to obtain a one-dimensional signal over time that depicts the average equivalent optical marking displacement across each image frame for a given physical displacement.

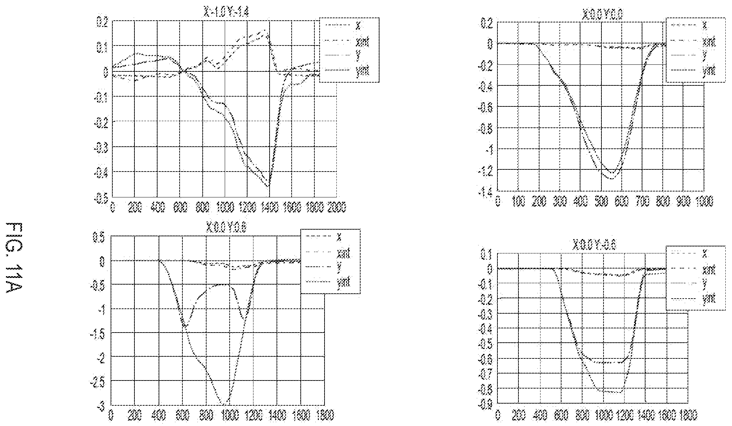

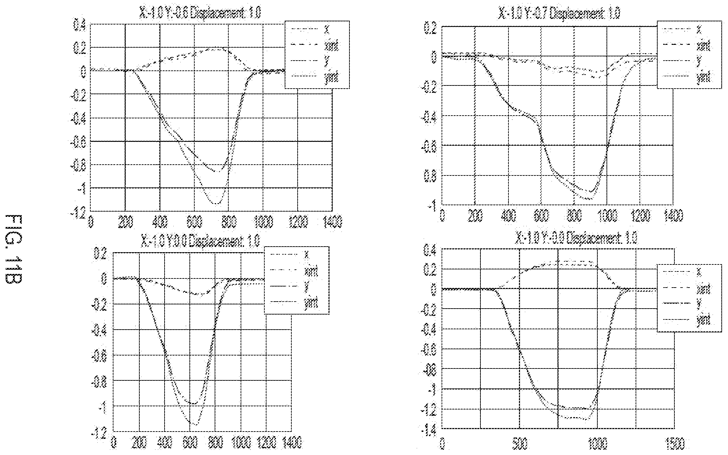

FIG. 11A provides graphs of illustrative displacement signals in pixel units (y axis) as the surface displacement system 108 is displaced in the range of 10-100s of microns over time (x axis) using a simple micrometer for the embodiments shown in FIGS. 2 and 3. FIG. 11B provides graphs of illustrative displacement signals in pixel units (y axis) as the surface displacement system 108 is displaced in the range of 10-100s of microns over time (x axis) using a simple micrometer for the embodiments shown in FIGS. 4 and 5. These graphs provides a understanding of how the surface displacement is measured in the optomechanical system 100.

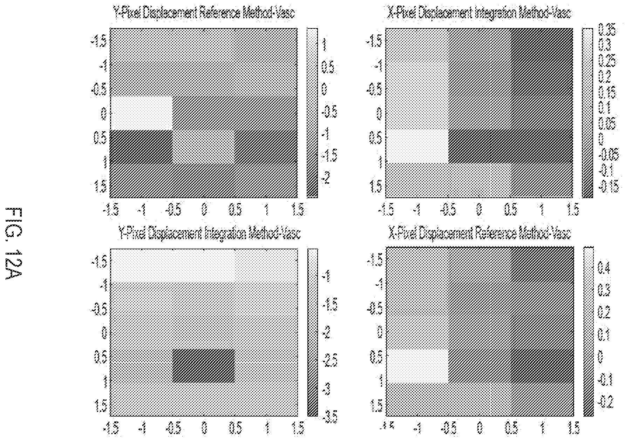

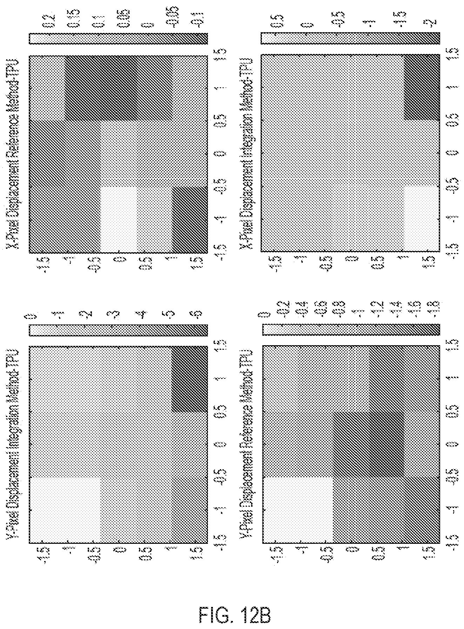

As can be seen in FIGS. 11A and 11B, the displacement of the optical markings 106 can be tracked over time. By tracking the temporal displacement of the optical markings 106, over an area of the image (e.g. 40mm x 30mm) provides the ability to map the temporal displacement of each pixels in a given image. FIGS. 12A and 12B depicts the surface displacement plots by measuring the maximum displacement of optical markings 106 at a given pixel for the embodiments shown in FIGS. 2 and 3 and FIGS. 4 and 5 respectively. One can generate surface displacement plots for every pixel for each point in time (e.g. 30ms) providing a corresponding high temporal resolution displacement information. This spatiotemporal displacement information can be used to locate and monitor the pulsation in the artery which in turn can be used to monitor the hemodynamic status of the patient. The high temporal resolution allows the optical system 102 to identify even sub-pixel movements due to the displacement of optical markings 106 which can be crucial to identify physiologically important parameters such as dicrotic notch in the blood pressure signal.

While various illustrative embodiments incorporating the principles of the present teachings have been disclosed, the present teachings are not limited to the disclosed embodiments. Instead, this application is intended to cover any variations, uses, or adaptations of the present teachings and use its general principles. Further, this application is intended to cover such departures from the present disclosure as come within known or customary practice in the art to which these teachings pertain.

In the above detailed description, reference is made to the accompanying drawings, which form a part hereof. In the drawings, similar symbols typically identify similar components, unless context dictates otherwise. The illustrative embodiments described in the present disclosure are not meant to be limiting. Other embodiments may be used, and other changes may be made, without departing from the scope of the subject matter presented herein. It will be readily understood that various features of the present disclosure, as generally described herein, and illustrated in the Figures, can be arranged, substituted, combined, separated, and designed in a wide variety of different configurations, all of which are explicitly contemplated herein.

The present disclosure is not to be limited in terms of the particular embodiments described in this application, which are intended as illustrations of various features. Many modifications and variations can be made without departing from its scope, as will be apparent to those skilled in the art. Functionally equivalent methods and apparatuses within the scope of the disclosure, in addition to those enumerated herein, will be apparent to those skilled in the art from the foregoing descriptions. It is to be understood that this disclosure is not limited to particular methods, reagents, compounds, compositions or biological systems, which can, of course, vary. It is also to be understood that the terminology used herein is for the purpose of describing particular embodiments only, and is not intended to be limiting.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein are generally intended as "open" terms (for example, the term "including" should be interpreted as "including but not limited to," the term "having" should be interpreted as "having at least," the term "includes" should be interpreted as "includes but is not limited to," et cetera). While various compositions, methods, and devices are described in terms of "comprising" various components or steps (interpreted as meaning "including, but not limited to"), the compositions, methods, and devices can also "consist essentially of" or "consist of" the various components and steps, and such terminology should be interpreted as defining essentially closed-member groups.

In addition, even if a specific number is explicitly recited, those skilled in the art will recognize that such recitation should be interpreted to mean at least the recited number (for example, the bare recitation of "two recitations," without other modifiers, means at least two recitations, or two or more recitations). Furthermore, in those instances where a convention analogous to "at least one of A, B, and C, et cetera" is used, in general such a construction is intended in the sense one having skill in the art would understand the convention (for example, "a system having at least one of A, B, and C" would include but not be limited to systems that have A alone, B alone, C alone, A and B together, A and C together, B and C together, and/or A, B, and C together, et cetera). In those instances where a convention analogous to "at least one of A, B, or C, et cetera" is used, in general such a construction is intended in the sense one having skill in the art would understand the convention (for example, "a system having at least one of A, B, or C" would include but not be limited to systems that have A alone, B alone, C alone, A and B together, A and C together, B and C together, and/or A, B, and C together, et cetera). It will be further understood by those within the art that virtually any disjunctive word and/or phrase presenting two or more alternative terms, whether in the description, sample embodiments, or drawings, should be understood to contemplate the possibilities of including one of the terms, either of the terms, or both terms. For example, the phrase "A or B" will be understood to include the possibilities of "A" or "B" or "A and B."

In addition, where features of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, et cetera. As a non-limiting example, each range discussed herein can be readily broken down into a lower third, middle third and upper third, et cetera. As will also be understood by one skilled in the art all language such as "up to," "at least," and the like include the number recited and refer to ranges that can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

The term "about," as used herein, refers to variations in a numerical quantity that can occur, for example, through measuring or handling procedures in the real world; through inadvertent error in these procedures; through differences in the manufacture, source, or purity of compositions or reagents; and the like. Typically, the term "about" as used herein means greater or lesser than the value or range of values stated by 1/10 of the stated values, e.g., ±10%. The term "about" also refers to variations that would be recognized by one skilled in the art as being equivalent so long as such variations do not encompass known values practiced by the prior art. Each value or range of values preceded by the term "about" is also intended to encompass the embodiment of the stated absolute value or range of values. Whether or not modified by the term "about," quantitative values recited in the present disclosure include equivalents to the recited values, e.g., variations in the numerical quantity of such values that can occur, but would be recognized to be equivalents by a person skilled in the art.

Various of the above-disclosed and other features and functions, or alternatives thereof, may be combined into many other different systems or applications. Various presently unforeseen or unanticipated alternatives, modifications, variations or improvements therein may be subsequently made by those skilled in the art, each of which is also intended to be encompassed by the disclosed embodiments.

The functions and process steps herein may be performed automatically or wholly or partially in response to user command. An activity (including a step) performed automatically is performed in response to one or more executable instructions or device operation without user direct initiation of the activity.

## Claims

1. An optomechanical sensor system (100) for use with a patient, the system comprising:
a patient-worn surface displacement system (108) comprising:
a deformable surface (109) configured to be positioned against the patient adjacent to a superficial artery thereof,
a plurality of optical markings (106) disposed on the deformable surface;
an actuator (110) configured to apply a pressure to hold the surface displacement system against the patient such that spatiotemporal movement of the superficial artery causes corresponding movement of the deformable surface and the plurality of optical markings;
an illumination source to illuminate the plurality of optical markings;
an imaging system;
and
a computer system (114) arranged to be coupled to the imaging system (102), the computer system comprising a processor (116) and a memory (118), the memory storing instructions that, when executed by the processor, cause the computer system to:
receive, from the imaging system (102), a plurality of images of the plurality of optical markings,
determine spatiotemporal movement of the optical markings based on the received plurality of images, and
determine a cardiopulmonary parameter associated with the patient based on the determined spatiotemporal movement of the optical markings, **characterized in that** the imaging system (102) is positioned at an oblique angle with respect to the optical markings such that it is configured to visualize the plurality of optical markings at an oblique angle so as to increase the sensitivity of surface displacement detection in the images captured by the imaging system.

2. The optomechanical sensor system of claim 1, wherein the deformable surface (109) is selected from the group consisting of an elastomeric gel (120), a fluid-filled balloon (128), a membrane (130), and a combination thereof.

3. The optomechanical sensor system of claim 1, wherein the actuator (110) is selected from the group consisting of a piston (160) configured to be moved to adjust the pressure on the deformable surface, an inflatable bladder (128), an adjustable strap (126), a chamber (125) coupled to an air pump (142), and a combination thereof.

4. The optomechanical sensor system of claim 1, wherein the superficial artery comprises a radial artery.

5. The optomechanical sensor system of claim 1, wherein the cardiopulmonary parameter is selected from the group consisting of a heart rate, a blood pressure, a respiratory rate, and a combination thereof.

6. The optomechanical sensor system of claim 1, wherein the optical system is further comprising: a mirror system (131) configured to cause the imaging system to visualize the plurality of optical markings at the oblique angle.

7. The optomechanical sensor system of claim 1, further comprising: a housing containing the patient-worn surface displacement system and the imaging system.

8. The optomechanical sensor system of claim 1, wherein the imaging system is separate from the patient-worn surface displacement system.

9. The optomechanical sensor system of claim 1, wherein the plurality of optical markings comprise optically visible markings, or wherein the plurality of optical markings comprise fluorescent markings.

10. The optomechanical sensor system of claim 1, further comprising:
a clear layer situated between the imaging system (104) and the plurality of optical markings,
wherein the imaging system is configured to visualize the plurality of optical markings through the clear layer.

11. The optomechanical sensor system of claim 10, wherein the clear layer is selected from a clear plate (122) and a clear balloon.

12. A method of monitoring a cardiopulmonary parameter of a patient using the optomechanical system of claim 1, the method comprising:
affixing the surface displacement system adjacent to a superficial artery of the patient;
causing the actuator to apply the pressure to hold the surface displacement system against the patient such that spatiotemporal movement of the superficial artery causes corresponding movement of the deformable surface and the plurality of optical markings;
illuminating with the illumination source the plurality of optical markings;
capturing, via the imaging system, a plurality of images of the plurality of optical markings;
determining, via the computer system, spatiotemporal movement of the optical markings based on the captured plurality of images; and
determining, via the computer system, a cardiopulmonary parameter associated with the patient based on the determined spatiotemporal movement of the optical markings.

13. The method of claim 12, wherein the deformable surface is selected from the group consisting of an elastomeric gel, a fluid-filled balloon, a membrane, and a combination thereof and/or the actuator is selected from the group consisting of a piston configured to be moved to adjust the pressure on the deformable surface, an inflatable bladder (128), an adjustable strap, a chamber coupled to an air pump (142), and a combination thereof.

14. The method of claim 12, wherein the superficial artery comprises a radial artery.

15. The method of claim 12, wherein the cardiopulmonary parameter is selected from the group consisting of a heart rate, a blood pressure, a respiratory rate, or a combination thereof.

## Patentansprüche

1. Optomechanisches Sensorsystem (100) zur Verwendung bei einem Patienten, wobei das System umfasst:
ein vom Patienten getragenes Oberflächenverschiebungssystem (108), umfassend:
eine verformbare Oberfläche (109), die dazu konfiguriert ist, an dem Patienten benachbart zu einer oberflächlichen Arterie desselben positioniert zu werden,
eine Mehrzahl von optischen Markierungen (106), die auf der verformbaren Oberfläche angeordnet sind;
einen Aktuator (110), der dazu konfiguriert ist, einen Druck auszuüben, um das Oberflächenverschiebungssystem an dem Patienten so zu halten, dass eine raumzeitliche Bewegung der oberflächlichen Arterie eine entsprechende Bewegung der verformbaren Oberfläche und der Mehrzahl von optischen Markierungen bewirkt;
eine Beleuchtungsquelle zum Beleuchten der Mehrzahl von optischen Markierungen;
ein Bildgebungssystem;
und
ein Computersystem (114), das dazu eingerichtet ist, mit dem Bildgebungssystem (102) gekoppelt zu werden, wobei das Computersystem einen Prozessor (116) und einen Speicher (118) umfasst, wobei der Speicher Anweisungen speichert, die bewirken, wenn sie von dem Prozessor ausgeführt werden, dass das Computersystem:
von dem Bildgebungssystem (102) eine Mehrzahl von Bildern der Mehrzahl von optischen Markierungen empfängt,
die raumzeitliche Bewegung der optischen Markierungen basierend auf der empfangenen Mehrzahl von Bildern bestimmt, und
einen dem Patienten zugeordneten kardiopulmonalen Parameter basierend auf der bestimmten raumzeitlichen Bewegung der optischen Markierungen bestimmt,
**dadurch gekennzeichnet, dass** das Bildgebungssystem (102) in einem schrägen Winkel in Bezug auf die optischen Markierungen positioniert ist, so dass es dazu konfiguriert ist, die Mehrzahl von optischen Markierungen in einem schrägen Winkel zu visualisieren, um die Empfindlichkeit der Oberflächenverschiebungserkennung in den von dem Bildgebungssystem erfassten Bildern zu erhöhen.

2. Optomechanisches Sensorsystem nach Anspruch 1, wobei die verformbare Oberfläche (109) aus der Gruppe ausgewählt ist, die aus einem elastomeren Gel (120), einem fluidgefüllten Ballon (128), einer Membran (130) und einer Kombination davon besteht.

3. Optomechanisches Sensorsystem nach Anspruch 1, wobei der Aktuator (110) aus der Gruppe ausgewählt ist, die aus einem Kolben (160), der dazu konfiguriert ist, bewegt zu werden, um den Druck auf die verformbare Oberfläche einzustellen, einer aufblasbaren Blase (128), einem einstellbaren Riemen (126), einer mit einer Luftpumpe (142) gekoppelten Kammer (125) und einer Kombination davon besteht.

4. Optomechanisches Sensorsystem nach Anspruch 1, wobei die oberflächliche Arterie eine Radialarterie umfasst.

5. Optomechanisches Sensorsystem nach Anspruch 1, wobei der kardiopulmonale Parameter aus der Gruppe ausgewählt ist, die aus einer Herzfrequenz, einem Blutdruck, einer Atemfrequenz und einer Kombination davon besteht.

6. Optomechanisches Sensorsystem nach Anspruch 1, wobei das optische System ferner umfasst: ein Spiegelsystem (131), das dazu konfiguriert ist, zu bewirken, dass das Bildgebungssystem die Mehrzahl von optischen Markierungen in dem schrägen Winkel visualisiert.

7. Optomechanisches Sensorsystem nach Anspruch 1, ferner umfassend: ein Gehäuse, das das vom Patienten getragene Oberflächenverschiebungssystem und das Bildgebungssystem enthält.

8. Optomechanisches Sensorsystem nach Anspruch 1, wobei das Bildgebungssystem von dem vom Patienten getragenen Oberflächenverschiebungssystem getrennt ist.

9. Optomechanisches Sensorsystem nach Anspruch 1, wobei die Mehrzahl von optischen Markierungen optisch sichtbare Markierungen umfasst, oder wobei die Mehrzahl von optischen Markierungen fluoreszierende Markierungen umfasst.

10. Optomechanisches Sensorsystem nach Anspruch 1, ferner umfassend:
eine durchsichtige Schicht, die sich zwischen dem Bildgebungssystem (104) und der Mehrzahl von optischen Markierungen befindet,
wobei das Bildgebungssystem dazu konfiguriert ist, die Mehrzahl von optischen Markierungen durch die durchsichtige Schicht hindurch zu visualisieren.

11. Optomechanisches Sensorsystem nach Anspruch 10, wobei die durchsichtige Schicht aus einer durchsichtigen Platte (122) und einem durchsichtigen Ballon ausgewählt ist.

12. Verfahren zum Überwachen eines kardiopulmonalen Parameters eines Patienten unter Verwendung des optomechanischen Systems nach Anspruch 1, wobei das Verfahren umfasst:
Befestigen des Oberflächenverschiebungssystems benachbart zu einer oberflächlichen Arterie des Patienten;
Bewirken, dass der Aktuator den Druck ausübt, um das Oberflächenverschiebungssystem an dem Patienten so zu halten, dass eine raumzeitliche Bewegung der oberflächlichen Arterie eine entsprechende Bewegung der verformbaren Oberfläche und der Mehrzahl von optischen Markierungen bewirkt;
Beleuchten der Mehrzahl von optischen Markierungen mit der Beleuchtungsquelle;
Erfassen, über das Bildgebungssystem, einer Mehrzahl von Bildern der Mehrzahl von optischen Markierungen;
Bestimmen, über das Computersystem, einer raumzeitlichen Bewegung der optischen Markierungen, basierend auf der erfassten Mehrzahl von Bildern; und
Bestimmen, über das Computersystem, eines dem Patienten zugeordneten kardiopulmonalen Parameters basierend auf der bestimmten raumzeitlichen Bewegung der optischen Markierungen.

13. Verfahren nach Anspruch 12, wobei die verformbare Oberfläche aus der Gruppe ausgewählt ist, die aus einem elastomeren Gel, einem fluidgefüllten Ballon, einer Membran und einer Kombination davon besteht, und/oder der Aktuator aus der Gruppe ausgewählt ist, die aus einem Kolben, der dazu konfiguriert ist, bewegt zu werden, um den Druck auf die verformbare Oberfläche einzustellen, einer aufblasbaren Blase (128), einem einstellbaren Riemen, einer mit einer Luftpumpe (142) gekoppelten Kammer und einer Kombination davon besteht.

14. Verfahren nach Anspruch 12, wobei die oberflächliche Arterie eine Radialarterie umfasst.

15. Verfahren nach Anspruch 12, wobei der kardiopulmonale Parameter aus der Gruppe ausgewählt ist, die aus einer Herzfrequenz, einem Blutdruck, einer Atemfrequenz oder einer Kombination davon besteht.

## Revendications

1. Système de capteur optomécanique (100) pour utilisation avec un patient, le système comprenant :
un système de déplacement de surface porté par le patient (108) comprenant :
une surface déformable (109) configurée pour être positionnée contre le patient de manière adjacente à une artère superficielle de celui-ci,
une pluralité de repères optiques (106) disposés sur la surface déformable ;
un actionneur (110) configuré pour appliquer une pression afin de maintenir le système de déplacement de surface contre le patient de telle sorte que le mouvement spatio-temporel de l'artère superficielle provoque un mouvement correspondant de la surface déformable et de la pluralité de repères optiques ;
une source d'éclairage pour éclairer la pluralité de repères optiques ;
un système d'imagerie ;
et
un système informatique (114) agencé pour être couplé au système d'imagerie (102), le système informatique comprenant un processeur (116) et une mémoire (118), la mémoire stockant des instructions qui, lorsqu'elles sont exécutées par le processeur, amènent le système informatique à :
recevoir, à partir du système d'imagerie (102), une pluralité d'images de la pluralité de repères optiques,
déterminer le mouvement spatio-temporel des repères optiques sur la base de la pluralité d'images reçues, et
déterminer un paramètre cardiopulmonaire associé au patient sur la base du mouvement spatio-temporel déterminé des repères optiques,
**caractérisé en ce que** le système d'imagerie (102) est positionné à un angle oblique par rapport aux repères optiques de manière à être configuré pour visualiser la pluralité de repères optiques à un angle oblique afin d'augmenter la sensibilité de la détection du déplacement de surface dans les images capturées par le système d'imagerie.

2. Système de capteur optomécanique selon la revendication 1, dans lequel la surface déformable (109) est choisie dans le groupe constitué par un gel élastomère (120), un ballon rempli de fluide (128), une membrane (130) et une combinaison de ceux-ci.

3. Système de capteur optomécanique selon la revendication 1, dans lequel l'actionneur (110) est choisi dans le groupe constitué par un piston (160) configuré pour être déplacé afin d'ajuster la pression sur la surface déformable, une vessie gonflable (128), une sangle réglable (126), une chambre (125) couplée à une pompe à air (142) et une combinaison de ceux-ci.

4. Système de capteur optomécanique selon la revendication 1, dans lequel l'artère superficielle comprend une artère radiale.

5. Système de capteur optomécanique selon la revendication 1, dans lequel le paramètre cardiopulmonaire est choisi dans le groupe constitué par une fréquence cardiaque, une pression artérielle, une fréquence respiratoire et une combinaison de celles-ci.

6. Système de capteur optomécanique selon la revendication 1, dans lequel le système optique comprend en outre : un système de miroirs (131) configuré pour amener le système d'imagerie à visualiser la pluralité de repères optiques à l'angle oblique.

7. Système de capteur optomécanique selon la revendication 1, comprenant en outre : un boîtier contenant le système de déplacement de surface porté par le patient et le système d'imagerie.

8. Système de capteur optomécanique selon la revendication 1, dans lequel le système d'imagerie est séparé du système de déplacement de surface porté par le patient.

9. Système de capteur optomécanique selon la revendication 1, dans lequel la pluralité de repères optiques comprend des repères optiquement visibles, ou dans lequel la pluralité de repères optiques comprend des repères fluorescents.

10. Système de capteur optomécanique selon la revendication 1, comprenant en outre :
une couche transparente située entre le système d'imagerie (104) et la pluralité de repères optiques,
dans lequel le système d'imagerie est configuré pour visualiser la pluralité de repères optiques à travers la couche transparente.

11. Système de capteur optomécanique selon la revendication 10, dans lequel la couche transparente est choisie parmi une plaque transparente (122) et un ballon transparent.

12. Procédé de surveillance d'un paramètre cardiopulmonaire d'un patient à l'aide du système optomécanique selon la revendication 1, le procédé comprenant :
la fixation du système de déplacement de surface de manière adjacente à une artère superficielle du patient ;
le fait d'amener l'actionneur à appliquer la pression afin de maintenir le système de déplacement de surface contre le patient de telle sorte que le mouvement spatio-temporel de l'artère superficielle provoque un mouvement correspondant de la surface déformable et de la pluralité de repères optiques ;
l'éclairage de la pluralité de repères optiques à l'aide de la source d'éclairage ;
la capture, via le système d'imagerie, d'une pluralité d'images de la pluralité de repères optiques ;
la détermination, via le système informatique, du mouvement spatio-temporel des repères optiques sur la base de la pluralité d'images capturées ; et
la détermination, via le système informatique, d'un paramètre cardiopulmonaire associé au patient sur la base du mouvement spatio-temporel déterminé des repères optiques.

13. Procédé selon la revendication 12, dans lequel la surface déformable est choisie dans le groupe constitué par un gel élastomère, un ballon rempli de fluide, une membrane et une combinaison de ceux-ci, et/ou l'actionneur est choisi dans le groupe constitué par un piston configuré pour être déplacé afin d'ajuster la pression sur la surface déformable, une vessie gonflable (128), une sangle réglable, une chambre couplée à une pompe à air (142) et une combinaison de ceux-ci.

14. Procédé selon la revendication 12, dans lequel l'artère superficielle comprend une artère radiale.

15. Procédé selon la revendication 12, dans lequel le paramètre cardiopulmonaire est choisi dans le groupe constitué par une fréquence cardiaque, une pression artérielle, une fréquence respiratoire ou une combinaison de celles-ci.
